# EUROPEAN PATENT APPLICATION

(11) **EP 3 045 464 A1**
(43) Date of publication of application: **20.07.2016**
(21) Application number: 14844510.9
(22) Date of filing: 09.09.2014
(51) Int. Cl.: C07F 9/6561, A61K 31/675, C07D 487/04, A61P 9/10, A61P 11/00, A61P 25/16, A61P 25/28, A61P 27/02, A61P 27/06, A61P 35/00, A61P 35/02, A61P 43/00

(54) **METHOD OF PRODUCING PYRAZINO[2,1-C][1,2,4]TRIAZINE COMPOUND**

(30) Priority: 11.09.2013 JP 2013188361
(71) Applicant: PRISM Pharma Co., Ltd., Kanagawa 226-8510 (JP)
(72) Inventor: ISOMURA Minetaka, Kamisu-shi Ibaraki 314-0255 (JP); KOSAKA Yuki, Kamisu-shi Ibaraki 314-0255 (JP); NAGAI Mitsuo, Tsukuba-shi Ibaraki 300-2635 (JP); ODAGAMI Takenao, Yokohama-shi Kanagawa 226-8510 (JP); KOUJI Hiroyuki, Yokohama-shi Kanagawa 226-8510 (JP)
(74) Representative: Duckworth, Timothy John
(86) International application number: PCT/JP2014/073821
(87) International publication number: WO 2015/037587

(57) **Abstract**

A method of producing 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, including a step of adding a reaction mixture 1 containing (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide, triethylamine and a solvent to a reaction mixture 2 containing a phosphorylating agent and a solvent.

## Description

### Technical Field

The present invention relates to a production method of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate (hereinafter to be referred to as compound 1).

### Background Art

Compound 1 described in patent document 1 is a compound that inhibits the TCF4/β-Catenin transcription pathway by inhibiting CREB binding protein (CBP) in the Wnt signal transduction pathway, and is expected to treat various cancers (e.g., lung cancer, breast cancer, stomach cancer, pancreatic cancer, liver cancer, uterine cancer, ovarian cancer, glioma, melanoma, rectal colon cancer, lymphoma, leukemia), restenosis relating to angioplasty, angiogenesis abnormality, polycystic kidney, tuberous sclerosis, Alzheimer's disease, neurodegenerative diseases (e.g., glaucoma, macular degeneration, Parkinson's disease, Alzheimer's disease) and fibrosis (e.g., idiopathic pulmonary fibrosis).

### Document List

### patent document

patent document 1: WO 2009/148192

### Summary of the Invention

### Problems to be Solved by the Invention

In patent document 1, compound 1 is produced by adding phosphoryl chloride to a solution of (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide (hereinafter to be referred to as compound 2) and triethylamine in tetrahydrofuran to allow for reaction.

However, an industrially superior production method affording compound 1 at a higher yield and a higher purity than by the production method disclosed in patent document 1 has been demanded. Therefore, an object of the present invention is to provide an industrially more superior production method that affords compound 1 at a higher yield and a higher purity. Means of Solving the Problems

The present inventors have conducted intensive studies and found a production method that affords compound 1 at a higher yield and a higher purity, and completed the present invention.

That is, the present invention provides the following [1] - [9].
[1] A method of producing 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, comprising a step of adding a reaction mixture 1 comprising (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide, an organic base and a solvent to a reaction mixture 2 comprising a phosphorylating agent and a solvent.
[2] The production method of [1], further comprising a step of mixing a solution of a mixture of the above-mentioned reaction mixture 1 and the above-mentioned reaction mixture 2 and an inorganic base.
[3] The production method of [1], comprising adding a solution of a mixture of the above-mentioned reaction mixture 1 and the above-mentioned reaction mixture 2 to a reaction mixture 3 comprising an inorganic base and water.
[4] The production method of [2] or [3], wherein the above-mentioned inorganic base is sodium hydroxide.
[5] The production method of any one of items [1] - [4], wherein the solvent in the above-mentioned reaction mixture 1 is tetrahydrofuran.
[6] The production method of any one of items [1] - [5], wherein the solvent in the above-mentioned reaction mixture 2 is tetrahydrofuran.
[7] The production method of any one of items [1] - [6], wherein a water content of the above-mentioned reaction mixture 1 is less than 0.5%.
[8] The production method of any one of items [1] - [7], wherein a water content of the above-mentioned reaction mixture 1 is less than 0.1%.
[9] The production method of any one of items [1] - [8], wherein the above-mentioned reaction mixture 2 is cooled to -15 to -10°C when the above-mentioned reaction mixture 1 is added to the above-mentioned reaction mixture 2.

### Effect of the Invention

According to the present invention, the amounts of a phosphorylating agent and a reagent such as organic base and the like to be used can be reduced by adding the reaction mixture 1 to the reaction mixture 2. According to the present invention, moreover, generation of impurities such as dimer and the like can be suppressed, and compound 1 having a higher yield and a higher purity can be obtained. According to the present invention, moreover, environmental burden can also be reduced.

### Description of Embodiments

The present invention is explained in detail in the following by showing the definition and the like of the symbols, terms and the like used in the present specification.

The production method of the present invention is explained below.

The present invention comprises a step of adding reaction mixture 1 comprising compound 2, an organic base and a solvent to reaction mixture 2 comprising a phosphorylating agent and a solvent (step A), and a step of mixing a solution of a mixture of reaction mixture 1 and reaction mixture 2 and an inorganic base (step B).

### (step A)

In step A, reaction mixture 1 comprising compound 2, an organic base and a solvent is added to reaction mixture 2 comprising a phosphorylating agent and a solvent.

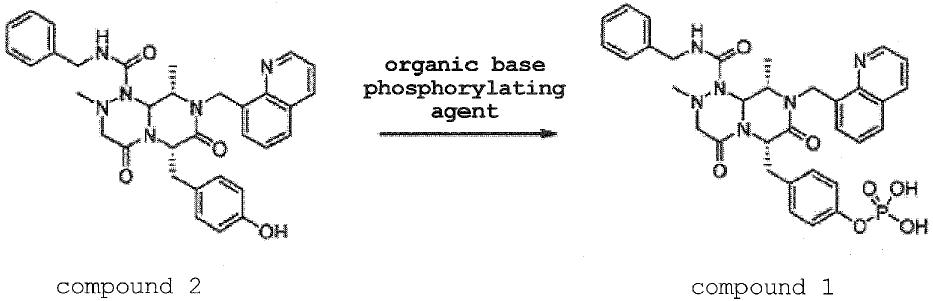

A solvent to be used for the reaction mixture 1 is not particularly limited as long as it dissolves compound 2 and an organic base, and does not inhibit the reactions to be performed thereafter. Examples of such solvent include tetrahydrofuran, dimethoxyethane, t-butyl methyl ether, 2-methyltetrahydrofuran, toluene, heptane, and ethyl acetate, preferably, tetrahydrofuran.

The amount of the solvent to be used for reaction mixture 1 is 5 - 20 mL, preferably 8 - 12 mL, per mass 1 g of compound 2.

The water content of reaction mixture 1 is preferably less than 0.5%, more preferably less than 0.1%. The water content of reaction mixture 1 can be measured, for example, by the Karl Fischer method. When the water content of reaction mixture 1 is within the above-mentioned range, compound 1 can be obtained at a higher yield.

When the water content of reaction mixture 1 is higher than the above-mentioned range, the water content can be lowered by repeating an azeotropic distillation operation with toluene, tetrahydrofuran and the like. Preferred is tetrahydrofuran since it can dissolve reagents used in step A.

The organic base to be used for reaction mixture 1 is not particularly limited as long as it is an organic base generally used in the field of organic chemistry. As such organic base, preferred is trialkylamine and particularly preferred is triethylamine. The number of moles of the organic base to be used for step A is not less than 1 equivalent, preferably 1 - 8 equivalents, more preferably 1.5 - 5 equivalents, based on the number of moles of compound 2 as 1 equivalent.

The temperature of reaction mixture 1 is preferably 10 - 40°C, more preferably 20 - 30°C.

A solvent to be used for the reaction mixture 2 is not particularly limited as long as it dissolves phosphorylating agents, and does not inhibit the reactions to be performed thereafter. Examples of such solvent include tetrahydrofuran, dimethoxyethane, t-butyl methyl ether, 2-methyltetrahydrofuran, toluene, heptane, and ethyl acetate, preferably, tetrahydrofuran.

The phosphorylating agent is not particularly limited as long as it phosphorylates compound 2 to give compound 1. Examples of such phosphorylating agent include phosphoric acid halides such as phosphoric acid chloride, phosphoric acid bromide and the like; bis(dimethylamino)phosphoryl chloride, and bis(diethylamino)phosphoryl chloride. The phosphorylating agent is preferably phosphoric acid chloride, more preferably phosphoryl chloride. The number of moles of the phosphorylating agent to be used for step A is not less than 1 equivalent, preferably 1 - 8 equivalents, more preferably 1.5 - 5 equivalents, based on the number of moles of compound 2 as 1 equivalent.

The temperature of reaction mixture 2 in use is preferably not more than 0°C, more preferably -15 to -10°C. In such temperature range, generation of impurity tends to be suppressed more, which is preferable from the aspects of the control of reaction temperature and economic efficiency.

Step A is performed by adding reaction mixture 1 to reaction mixture 2. For the addition of reaction mixture 1, an apparatus for adding small amounts such as a syringe pump, a dropping funnel and the like is used, whereby a desired reaction with less generation of impurity such as dimer and the like can be achieved.

The reaction time is preferably 5 min - 5 hr, more preferably 15 min - 1 hr, after completion of the addition of reaction mixture 1 to reaction mixture 2.

### (step B)

Step B comprises a step of mixing a solution of a mixture of reaction mixture 1 and reaction mixture 2 and an inorganic base (step B1), a step of washing the solution obtained in step B1 (mixture 1) with an organic solvent (step B2), and a step of adding an acid to mixture 2 obtained after the above-mentioned washing to precipitate compound 1 (step B3).

In step B1, a solution of a mixture of reaction mixture 1 and reaction mixture 2 is mixed with an inorganic base.

The inorganic base to be used in step B1 is not particularly limited as long as it decomposes the phosphorylating agent remained in step A. Examples of such inorganic base include sodium hydroxide, potassium hydroxide, sodium hydrogen carbonate, and sodium carbonate, preferably sodium hydroxide. When the inorganic base is sodium hydroxide, foaming during decomposition of the phosphorylating agent can be suppressed.

The inorganic base to be used in step B1 is preferably used in the form of reaction mixture 3 comprising the inorganic base and water. Using the inorganic base in the form of reaction mixture 3, heat generation associated with the decomposition of the phosphorylating agent can be suppressed, uniform mixing can be performed, and the remainig phosphorylating agent can be decomposed more efficiently.

Moreover, the method of adding mixture 1 to reaction mixture 3 can further suppress generation of impurities such as dimer and the like, and compound 1 can be obtained at higher purity and yield.

The temperature of mixture 1 when mixture 1 is mixed with an inorganic base is preferably not more than 15°C, more preferably 5 - 10°C.

The amount of an inorganic base to be used in step B1 is not particularly limited as long as it can decompose the phosphorylating agent remained in the reaction mixture in step A. The amount of such inorganic base is preferably 1 - 20 equivalents, more preferably 1 - 10 equivalents, based on the number of moles of the phosphorylating agent as 1 equivalent.

In step B2, mixture 1 is washed with an organic solvent.

The organic solvent used for washing in step B2 is not particularly limited as long as it is an organic solvent that does not react with compound 1. Such organic solvent is preferably ethyl acetate. Dimers can be removed more efficiently by washing with ethyl acetate.

In step B3, an acid is added to mixture 2, obtained after step B2, to precipitate compound 1.

Mixture 2 may be diluted with an organic solvent before addition of an acid to mixture 2. As such organic solvent, ethanol can be mentioned. The amount of such organic solvent can be appropriately adjusted to prevent a decrease in the precipitation rate of compound 1.

An acid to be added to mixture 2 is not particularly limited as long as it releases proton. Examples of such acid include hydrochloric acid and sulfuric acid, preferably hydrochloric acid.

In step B3, mixture 2 is preferably agitated during addition of an acid to mixture 2. By agitating mixture 2, the acid can be diffused more uniformly, and precipitation of compound 1 in a bulky state can be suppressed.

Compound 1 can be obtained in step B3 by collecting the precipitate by filtration and drying same.

Compound 1 may be dried by heating under reduced pressure or with a hot air.

One embodiment of the present invention may include a step of reacting N-benzyloxycarbonyl-L-alanine, carbonyl diimidazole with N-methoxy-N-methylamine hydrochloride (step C), a step of reacting (S)-benzyl 1-(methoxy(methyl)amino)-1-oxopropan-2-ylcarbamate with sodium bis(2-methoxyethoxy)aluminum hydride (step D), a step of reacting (S)-benzyl 1-oxopropan-2-ylcarbamate with triethyl orthoformate (step E), or a step of obtaining 2-(2-(benzylcarbamoyl)-1-methylhydrazinyl)acetic acid by a one-pot reaction using ethyl bromoacetate, monomethylhydrazine and benzyl isocyanate as starting materials (step F).

When steps C - F are included, a decrease in the optical purity tends to be suppressed, the amounts of reagents and solvents to be used can be reduced as the number of steps decreases, and an economically advantageous method can be afforded. Steps C - F are explained below.

### (step C)

In step C, N-benzyloxycarbonyl-L-alanine, carbonyl diimidazole and N-methoxy-N-methylamine hydrochloride are reacted.

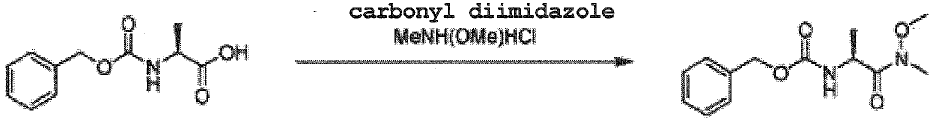

According to step C, (S)-benzyl 1-(methoxy(methyl)amino)-1-oxopropan-2-ylcarbamate can be obtained at a high yield by using an industrially practical reagent, carbonyl diimidazole.

Step C can be performed in a solvent or without solvent, wherein organic solvents are not particularly limited as long as they do not influence the reaction. Examples of such solvent include acetonitrile, tetrahydrofuran, dimethoxyethane, t-butyl methyl ether, 2-methyltetrahydrofuran, toluene, heptane, and ethyl acetate, preferably acetonitrile.

The reaction temperature in step C is preferably 0 - 30°C, more preferably 20 - 25°C.

### (step D)

In step D, (S)-benzyl 1-(methoxy(methyl)amino)-1-oxopropan-2-ylcarbamate is reacted with sodium bis(2-methoxyethoxy)aluminum hydride.

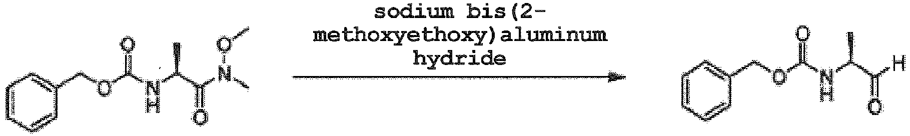

According to step D, (S)-benzyl 1-oxopropan-2-ylcarbamate can be obtained using an industrially practical reagent, sodium bis(2-methoxyethoxy)aluminum hydride, and a decrease in the optical purity can be suppressed further.

The solvents to be used in step D are not particularly limited as long as they are organic solvents that do not influence the reaction. Examples of such solvent include diisopropyl ether, and tetrahydrofuran, preferably tetrahydrofuran.

The reaction temperature in step D is preferably -40 to 0°C, more preferably -25 to -10°C.

### (step E)

In step E, (S)-benzyl 1-oxopropan-2-ylcarbamate is reacted with triethyl rthoformate.

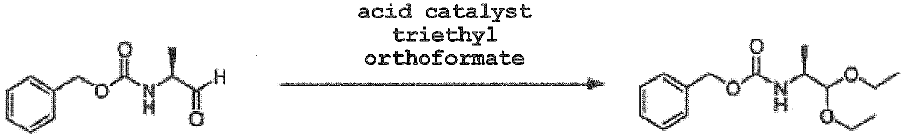

According to step E, (S)-benzyl 1,1-diethoxypropan-2-ylcarbamate can be obtained at a high yield, and a decrease in the optical purity of (S)-benzyl 1,1-diethoxypropan-2-ylcarbamate obtained in step E can be suppressed further.

In step E, an acid catalyst is preferably added. Such acid catalyst is preferably an acid having a proton, more preferably an acid addition salt such as tertiary amine, pyridine and the like. Examples of the acid addition salt of tertiary amine, pyridine and the like include pyridinium p-toluenesulfonate. When the acid catalyst is an acid addition salt of tertiary amine, pyridine and the like, a decrease in the optical purity of (S)-benzyl 1,1-diethoxypropan-2-ylcarbamate obtained in step E can be further suppressed.

The amount of the acid catalyst to be used in step E is 0.3 - 0.01 equivalent, preferably 0.1 - 0.03 equivalent, based on the number of moles of (S)-benzyl 1-oxopropan-2-ylcarbamate as 1 equivalent.

The reaction temperature in step E is preferably 0 - 30°C, more preferably 15 - 25°C. In such temperature range, a decrease in the optical purity of (S)-benzyl 1,1-diethoxypropan-2-ylcarbamate obtained in step E tends to be further suppressed.

In step F, 2-(2-(benzylcarbamoyl)-1-methylhydrazinyl)acetic acid is obtained by a one-pot reaction using ethyl bromoacetate, monomethylhydrazine and benzyl isocyanate as starting materials.

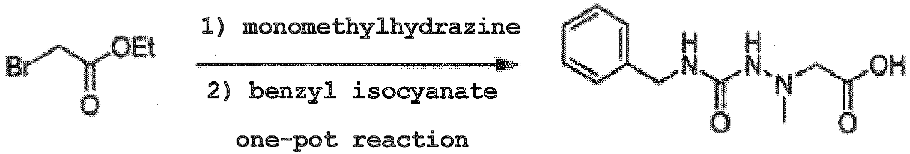

According to step F, ethyl bromoacetate is reacted with methylhydrazine. After that benzyl isocyanate is added to the obtained reaction mixture, and the mixture is further hydrolyzed to give 2-(2-(benzylcarbamoyl)-1-methylhydrazinyl)acetic acid at a high yield without purification in middle of a step.

Production by one-pot reaction can reduce the necessary amount of a solvent to be used for each reaction and purification. Therefore, step F is preferable from the aspects of the reduction of environmental burden and cost. When production by one-pot reaction is possible, particularly, reagents, solvents and the like necessary for purification in each step of a conventional method are no longer necessary, and 2-(2-(benzylcarbamoyl)-1-methylhydrazinyl)acetic acid can be efficiently produced in a shorter time.

### Examples

While the Examples of the present invention are described in detail in the following, the Examples are not limitative. In addition, "eq" appearing in the Examples shows the number of moles (mol) used per 1 mol of component indicated as "1 eq", and "vol" shows the volume (mL) used per 1 g of component indicated as "1 eq". The optical purity is indicated as enantiomeric excess (%ee).

### Example 1

### Production method of compound 1

Anhydride of compound 2 (10.2 g, 1 eq.) described in WO 2009/148192 was placed in a eggplant-shaped flask 1, tetrahydrofuran (70 mL, 7 vol) and triethylamine (9.6 mL, 4.0 eq) were added at room temperature, and the obtained mixture was stirred (hereinafter to be referred to as reaction mixture 1). After stirring, the water content of reaction mixture 1 was measured by the Karl Fischer method to confirm that the water content was less than 500 ppm. Tetrahydrofuran (80 mL, 8 vol) was placed in a different eggplant-shaped flask 2, cooled to -15°C, and phosphoryl chloride (3.2 mL, 2.0 eq) was added to give a mixture (hereinafter to be referred to as reaction mixture 2). Using a syringe pump, reaction mixture 1 was added dropwise to the eggplant-shaped flask containing reaction mixture 2 over 70 min while maintaining the inside temperature at -17.1 to -16.3°C. The eggplant-shaped flask containing reaction mixture 1 was washed with tetrahydrofuran (5 mL, 0.5 vol), and the washing was added to eggplant-shaped flask 2. After stirring for 35 min, cease of the reaction was confirmed by HPLC.

5N Aqueous sodium hydroxide solution (25.4 mL, 7.35 eq) and water (224.6 mL, 22.5 vol) were placed in eggplant-shaped flask 3, and cooled with ice water (hereinafter to be referred to as reaction mixture 3). A mixture of reaction mixture 1 and reaction mixture 2 in eggplant-shaped flask 2 was added dropwise to reaction mixture 3 by using a Teflon cannula over 5 min while maintaining the inside temperature at 3.6 - 12.2°C. After completion of the dropwise addition, the mixture was stirred at outer temperature 6°C for 65 hr, and the reaction mixture was warmed to room temperature. Water (27.5 mL, 2.75 vol) was added to completely dissolve an inorganic salt (pH=9.7, liquid amount 435 mL, 43.5 vol). Ethyl acetate (100 mL, 10 vol) was added to the obtained solution (about 20% of the solution was used in Reference Example 8 to prepare crystal of compound 1 hydrate (seed crystal), and the rest of the solution was used) and the mixture was shaken in a separtion funnel, stood and the top layer was discarded. This operation was repeated 4 times to give aqueous solution containing compound 1 (liquid amount 274 mL, 27.4 vol).

Ethanol (65 mL, 6.84 vol) was added to an aqueous solution containing compound 1 (260 mL, corresponding to 9.5 g of compound 2) (pH=8.0). 37 mass % hydrochloric acid (total amount 3.14 mL, 0.33 vol) was slowly added dropwise to the obtained solution while cooling with a coolant at 20°C, then hydrate of compound 1 (111.35 mg) obtained in the below-mentioned Reference Example 8 was added thereto. After confirming an increase in the precipitate, the mixture was further stirred for about 20 min. Thereafter, the mixture was further acidified (pH=4.0) with 37 mass % hydrochloric acid (0.29 mL, 0.03 vol), gradually cooled to an inside temperature of -15°C over 5 hr, and the mixture was stirred for 11 hr. The mother liquor was heated to around 20°C, 37 mass % hydrochloric acid (2.28 mL, 0.24 vol) was added (pH=2.3), and the mixture was gradually cooled to an inside temperature of -10°C over for 4 hr, and stirred for about 15 hr. The matured mother liquor was suction filtered, and the obtained precipitate was washed with cold water - ethanol (volume ratio 8:2, 28.5 mL, 3 vol), cold water (47.5 mL, 5 vol), cold water - ethanol (volume ratio 8:2, 28.5 mL, 3 vol) in this order. Thereafter, the precipitate was dried under reduced pressure at 45°C for about 8 hr to give hydrate of compound 1 (yield 9.4 g, 87%, converted to yield of compound 1).
¹H-NMR (600 MHz, METHANOL-d₄) δ (ppm): 1.15 (d, J=6 Hz, 3H), 2.65 (s, 3H), 3.12 (d, J=18 Hz, 1H), 3.35 (d, J=7 Hz, 2H), 3.48 (d, J=18 Hz, 1H), 4.15 (m, 1H), 4.32 (d, J=15 Hz, 1H), 4.40 (d, J=15 Hz, 1H), 5.33 (d, J=16 Hz, 1H), 5.41 (d, J=16 Hz, 1H), 5.44 (d, J=7 Hz, 1H), 5.64 (d, J=10 Hz, 1H), 7.07 (dd, J=9, 1 Hz, 2H), 7.15 (d, J=9 Hz, 2H), 7.24 (t, J=7 Hz, 1H), 7.27 (d, J=7 Hz, 2H), 7.34 (t, J=8 Hz, 2H), 7.55 (dd, J=8, 4 Hz, 1H), 7.60 (brd, J=6 Hz, 1H), 7.62 (dd, J=8, 7 Hz, 1H), 7.88 (dd, J=8, 1 Hz, 1H), 8.38 (dd, J=8, 2 Hz, 1H), 8.90 (dd, J=4, 2 Hz, 1H).

According to the production method of Example 1, generation of impurity could be suppressed, and the yield was 87% which was higher than the yield (60%) described in Example 1-7 of patent document 1. In addition, the production method of Example 1 can produce compound 1 in a larger amount than by the production method described in patent document 1.

### Reference Example 1

### Synthesis of (S)-benzyl 1-(methoxy(methyl)amino)-1-oxopropan-2-ylcarbamate

N-benzyloxycarbonyl-L-alanine (9.8 g, 43.9 mmol), N-methoxy-N-methylamine hydrochloride (1.1 eq), 1-ethyl-3-(3-dimethylaminopropyl)carbodiimide hydrochloride (10 g,1.2 eq) and N,N-dimethyl-4-aminopyridine (0.53 g, 0.1 eq) were dissolved in N,N-dimethylformamide (100 mL, 10 vol), and N,N-diisopropylethylamine (23 mL, 3 eq) was added dropwise at room temperature over 30 min. After stirring for 5 hr, 2N hydrochloric acid (200 mL) was added to the reaction mixture, and the mixture was extracted with ethyl acetate (200 mL). The extract was washed with 1N aqueous sodium hydroxide solution (200 mL) to remove unreacted starting materials. The obtained organic layer was washed with saturated brine, and dried over anhydrous magnesium sulfate. The solvent was evaporated under reduced pressure, and the obtained oil was crystallized from methyl t-butyl ether (20 mL) and n-heptane (100 mL). The obtained crystals were collected by filtration, washed with n-heptane (20 mL), dried under reduced pressure to give the title compound as a white solid (yield 4.74 g, 41%).

### Reference Example 2

N-benzyloxycarbonyl-L-alanine (5.0 g, 22.4 mmol,1 eq) was dissolved in acetonitrile (50 mL, 10 vol), and carbonyl diimidazole (2.4 g, 1.1 eq) in a powder form was added over several min while stirring the mixture at room temperature. After stirring for 30 min, N-methoxy-N-methylamine hydrochloride (1.1 eq) in a powder form was added over several min. After 1.5 hr, 1N hydrochloric acid (50 mL) was added to the reaction mixture, and the mixture was extracted with ethyl acetate (50 mL). The organic layer was washed with aqueous sodium hydrogen carbonate solution-brine (50 mL), and dried over anhydrous magnesium sulfate. The solvent was evaporated under reduced pressure, ethyl acetate (10 mL) was added to the obtained solid and dissolved at 60°C and crystallized from n-heptane (50 mL) at room temperature. The obtained crystals were collected by filtration, washed with n-heptane (20 mL), dried under reduced pressure to give the title compound as a white solid (yield 5.22 g, 88%).

### Reference Example 3

To a solution of N-benzyloxycarbonyl-L-alanine (100 g,1 eq) in acetonitrile (3 vol) was added dropwise at 15°C a suspension of carbonyl diimidazole (80 g, 1.1 eq) in acetonitrile (7 vol) over 15 min. (Foaming was observed, and the reaction temperature rose by about 4°C. Insoluble material was precipitated at 10 min after dropwise addition.) After stirring for 15 min, N-methoxy-N-methylamine hydrochloride (48 g, 1.1 eq) in a powder form was added over 5 min (heat generation was somewhat observed immediately after addition). After stirring at room temperature for 30 min, disappearance of starting materials was confirmed by TLC. 1N hydrochloric acid (1000 mL) was added to the reaction mixture, and the mixture was extracted with ethyl acetate (once with 500 mL, once with 300 mL). The extracted layer was washed with sodium hydrogen carbonate-brine (300 mL) and water (300 mL), the obtained organic layer was concentrated under reduced pressure and azeotropically distilled with dehydrating with ethanol to give the object crude product (150 g). The obtained crude product was dissolved in ethyl acetate (150 mL) at 60°C, crystallized from n-heptane (600 mL) with stirring, and the precipitated solid was collected by filtration to give the title compound (yield 94.6 g, 80%) as primary crystals. The title compound (yield 18.7 g, 15.7%) was obtained as secondary crystals from the mother liquor. The purity and optical purity of the primary crystals and secondary crystals were measured by HPLC to find the purity of>99.5% for the both and the optical purity of almost 100%ee for the both. The total yield of the primary crystals and the secondary crystals was 113.3 g, 95.2%.

### Reference Example 4

### Synthesis of (S)-benzyl 1,1-diethoxypropan-2-ylcarbamate

(S)-benzyl 1-(methoxy(methyl)amino)-1-oxopropan-2-ylcarbamate (5.0 g, 18.8 mmol) was dissolved in tetrahydrofuran (50 mL, 10 vol), and sodium bis(2-methoxyethoxy)aluminum hydride 70% toluene solution (6.8 mL, 1.6 eq) was added dropwise at -10°C with stirring over 30 min. After stirring for 30 min, 2N hydrochloric acid (100 mL) was added dropwise over 15 min, and the mixture was stirred at room temperature for 30 min. The reaction mixture was extracted with ethyl acetate (50 mL), and the organic layer was washed with 5% aqueous sodium hydrogen carbonate solution (50 mL)-water (50 mL). The solvent was evaporated under reduced pressure to give (S)-benzyl 1-oxopropan-2-ylcarbamate (3.82 g) as a crude product. Ethanol (25 mL), triethyl orthoformate (2 eq), and pyridinium p-toluenesulfonate (0.1 eq) were added to the residue and the mixture was stirred at room temperature for 18 hr. To the reaction mixture was added 5% aqueous sodium hydrogen carbonate solution (50 mL) and the mixture was extracted with n-heptane (50 mL, twice). The combined organic layer was washed with water, and the solvent was evaporated under reduced pressure (water was azeotropically distilled with toluene) to give the title compound as a colorless oil (yield 4.42 g, 84%, optical purity 99.6%ee).

### Reference Example 5

(S)-benzyl 1-(methoxy(methyl)amino)-1-oxopropan-2-ylcarbamate (90 g, 0.338 mol) was dissolved in tetrahydrofuran (5.5 vol), and sodium bis(2-methoxyethoxy)aluminum hydride (127 g,1.3 eq) was added dropwise at -20°C over 30 min while maintaining the inside temperature at not more than -5°C. After dropwise addition, the mixture was stirred at -20°C for 30 min and disappearance of starting materials was confirmed by TLC. 5N hydrochloric acid (500 mL) was stirred at -20°C and the reaction mixture was added dropwise thereto over 40 min. The reaction mixture was partitioned, and the aqueous layer was extracted with ethyl acetate (5.5 vol) and combined with the organic layer. The mixture was washed with 5% aqueous sodium hydrogen carbonate solution (4 vol) and water (4 vol) and the solvent was evaporated under reduced pressure. Water was azeotropically distilled with ethanol to give (S)-benzyl 1-oxopropan-2-ylcarbamate (76 g) as a crude product. Ethanol (5 vol), triethyl orthoformate (112 mL, 2 eq), and pyridinium p-toluenesulfonate (4.2 g, 0.05 eq) were added to the residue and the mixture was stirred for 2 days for acetalization. The reaction mixture was partitioned by adding n-heptane (5 vol) and 5% aqueous sodium hydrogen carbonate solution (5 vol) and the aqueous layer was extracted with n-heptane (5 vol, twice). The combined organic layer was washed with water (5 vol) and the solvent was evaporated under reduced pressure (water and excess triethyl orthoformate were azeotropically distilled with toluene) to give the title compound (yield 84 g, 88%, optical purity 99.8%ee).

### Reference Example 6

### Synthesis of (S)-1,1-diethoxypropan-2-amine

(S)-benzyl 1,1-diethoxypropan-2-ylcarbamate (65 g, 0.231 mol) was dissolved in ethanol (130 mL, 2 vol), 10% Pd/C (6.5 g, 50% wet) was added, and catalytic reduction was performed for 12 hr under 0.35 MPa hydrogen. The catalyst was filtered, and the filtrate was distilled using Vigreux rectifying column under the following conditions. NMR and GC of the compound obtained as the main distillate (yield 28.1 g, 83%) were measured to confirm that it was the title compound. distillation conditions
first distillate:>30°C/50 mmHg (heating temperature: 50°C)
middle distillate: 60°C/15 mmHg (heating temperature: 85°C) 1.84 g
main distillate: 60°C/15 mmHg (heating temperature: 85°C) 28.1 g

### Reference Example 7

### Synthesis of 2-(2-(benzylcarbamoyl)-1-methylhydrazinyl)acetic acid

Ethyl bromoacetate (32.7 g, 0.9 eq) was dissolved in tetrahydrofuran (180 mL), and a solution of monomethylhydrazine (10 g, 1 eq) and triethylamine (20.9 g, 0.95 eq) in water (25 mL)-ethanol (50 mL) was added dropwise at -20°C with stirring over 35 min while maintaining the inside temperature at not more than -4.6°C. After stirring for 90 min, the disappearance of monomethylhydrazine was confirmed, and benzylisocyanate (27.4 g, 0.95 eq) was added dropwise over 40 min while maintaining the inside temperature at not more than -1.8°C. The mixture was stirred for 90 min, and production of ethyl 2-(2-(benzylcarbamoyl)-1-methylhydrazinyl)acetate was confirmed. Successively, 5N aqueous sodium hydroxide solution (130 mL) was added dropwise to the reaction mixture. After completion of the dropwise addition, the mixture was stirred at 20°C for 14 hr, and disappearance of ethyl 2-(2-(benzylcarbamoyl)-1-methylhydrazinyl)acetate was confirmed. The reaction mixture was washed with isopropyl acetate (250 mL, 4 times) and methyl t-butyl ether (250 mL). The aqueous layer was acidified (pH= 2.5) with 5N hydrochloric acid (84 mL), and a seed crystal (100 mg) was added. After stirring at 5°C for 23 hr, the mixture was filtered, washed with cold ethanol (25 mL)-water (75 mL), and dried under reduced pressure at 50°C for 4 hr to give the title compound as a white solid (31.7 g) (yield with monomethylhydrazine as standard: 61.6%, yield with ethyl bromoacetate as standard: 68.5%).

### Reference Example 8

### Hydrate of compound 1

To compound 1 (149.72 mg) described in WO 2009/148192 was added 4.0 mL of ethanol/water mixed solution (volume ratio 1:1) and the mixture was stirred with heating and completely dissolved at about 93°C. The mixture was allowed to slowly cool to room temperature, and precipitation of a solid was confirmed. The precipitate was collected by filtration through a glass filter, washed with heptane, and air dried at 60°C to give the title compound (yield 111.35 mg) as a white solid.

## Claims

1. A method of producing 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate by reacting (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide with a phosphorylating agent, which comprises a step of adding a reaction mixture 1 comprising (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide, an organic base and a solvent to a reaction mixture 2 comprising a phosphorylating agent and a solvent.

2. The production method according to claim 1, further comprising a step of mixing a solution of a mixture of said reaction mixture 1 and said reaction mixture 2 and an inorganic base.

3. The production method according to claim 1, further comprising a step of adding a solution of a mixture of said reaction mixture 1 and said reaction mixture 2 to a reaction mixture 3 comprising an inorganic base and water.

4. The production method according to claim 2 or 3, wherein said inorganic base is sodium hydroxide.

5. The production method according to any one of claims 1 to 4, wherein the solvent in said reaction mixture 1 is tetrahydrofuran.

6. The production method according to any one of claims 1 to 5, wherein the solvent in said reaction mixture 2 is tetrahydrofuran.

7. The production method according to any one of claims 1 to 6, wherein a water content of said reaction mixture 1 is less than 0.5%.

8. The production method according to any one of claims 1 to 7, wherein a water content of said reaction mixture 1 is less than 0.1%.

9. The production method according to any one of claims 1 to 8, wherein said reaction mixture 2 is cooled to -15 to -10°C when said reaction mixture 1 is added to said reaction mixture 2.
